# EUROPEAN PATENT SPECIFICATION

(11) **EP 1 064 037 B1**
(45) Date of publication and mention of the grant of the patent: **28.07.2004**
(21) Application number: 99912263.3
(22) Date of filing: 04.03.1999
(51) Int. Cl.: A61M 5/315, A61M 5/31, A61L 31/00

(54) **SYRINGE COMPRISING A POLYMERIC SILICONE LUBRICATION**
SPRITZE MIT SCHMIERUNG AUS SILIKONPOLYMEREN
SERINGUES LUBRIFIEES PAR UN POLYMERE DE SILICONE

(30) Priority: 17.03.1998 US 78264 P; 17.03.1998 US 78266 P
(43) Date of publication of application: 03.01.2001
(73) Proprietor: Becton Dickinson and Company, Franklin Lakes, New Jersey 07417 (US)
(72) Inventor: LUBRECHT, Thea, E., Randolph, NJ 07869 (US)
(74) Representative: Selting, Günther, Dipl.-Ing.
(86) International application number: PCT/US1999/004667
(87) International publication number: WO 1999/047192

(56) References cited:
- EP-A- 0 092 383
- EP-A- 0 111 724
- EP-A- 0 570 978
- EP-A- 0 627 474
- EP-A- 0 651 005
- US-A- 4 806 430
- US-A- 5 061 252

## Description

### BACKGROUND OF THE INVENTION

This invention generally relates to a method of lubricating the chamber body of a drug delivery system and, more particularly, to a method of lubricating a sealing member and the chamber body with a polymeric silicone.

Many drug delivery systems, like syringes, pre-filled syringes, drug cartridges and needleless injectors include an interior chamber for receiving a medicament and a sealing member. The sealing member is usually slidable within the interior chamber and in a fluid-tight relationship with the walls forming the interior chamber.

The sealing member can take many forms, with two conventional forms being a stopper and an O-ring. The sealing members are often made of rubber or elastomeric materials. The interior chamber of many drug delivery systems is made of glass or plastic. The fluid-tight relationship between the sealing member and the wall forming the interior chamber provides a large resistance to movement of the sealing member within the interior chamber. Typically, this resistance has been reduced by pre-treating the walls of the interior chamber and the sealing member with a lubricating solution such as silicone. In the typical coating method, the sealing member is agitated with a solution of silicone, then removed from the silicone solution and placed in the interior chamber of a drug delivery system. Typically, the surface of the walls of the interior chamber have also been pre-treated with a silicone solution.

There are several disadvantages with the typical lubricating method. The major disadvantage is that the lubricant typically is only loosely adhered to the sealing member or the interior chamber. This loose adherence permits the lubricating solution to become deposited into a medicament loaded in the drug delivery system. In some instances, spheres of silicone have been found suspended within the medicament solution. Another disadvantage associated with prior systems is that they sometimes require coating both the sealing member and the interior chamber with a lubrication solution.

Therefore, a drug delivery system that is lubricated in a manner that prevents the lubricant from becoming deposited in the medicament is desirable. This invention includes lubricating the sealing member and the chamber material using techniques that prevent the lubricant from accumulating in the medicament.

US-A-5 061 252 column 7 suggests a syringe where the material contains lubricant components which are mixed with a plastic resin before forming the parts of a syringe. Column 9 is mentioned that silicone is a known lubricant.

### SUMMARY OF THE INVENTION

In general terms, this invention is a medicament delivery device. A chamber has a body made from plastic material that includes a lubricating substance within the plastic. The chamber includes an inner surface for receiving the medicament. A sealing member may be slidably received within the chamber and has an exterior surface that sealingly engages the inner surface of the chamber. The sealing member outer surface includes a lubricating silicone substance that is adhered to the outer surface of the sealing member by cross linked bonds between molecules of the lubricating silicone. The cross linked bonds preferably are formed by irradiating the sealing member after the lubricating silicone has been applied to the outer surface.

In the most preferred embodiment, the lubricating silicone on the sealing member comprises a polydimethyl siloxane having a viscosity of approximately 0.1 m² per second (100,000 centistokes).

The delivery device of this invention preferably is made by the method according to claim 9.

These and other features and advantages of this invention will become more apparent to those skilled in the art from the following detailed description of the presently preferred embodiments. The drawings that accompany the detailed description can be described as follows.

### BRIEF DESCRIPTION OF THE DRAWINGS

Figure 1 is a flowchart illustrating the steps of lubricating a sealing member according to the method of this invention.
Figure 2 is a schematic illustration of the processing steps of manufacturing a prefilled syringe assembly designed according to this invention.
Figure 3 is a side, cross-sectional view of a drug delivery cartridge.
Figure 4 is an exploded, side view of a syringe and a needle cannula.
Figure 5 is a cross-sectional, side view of a stopper and a plunger.
Figure 6 is an exploded, side view of a medicament cartridge that can be used with a needleless injector.

### DETAILED DESCRIPTION OF THE PREFERRED EMBODIMENT

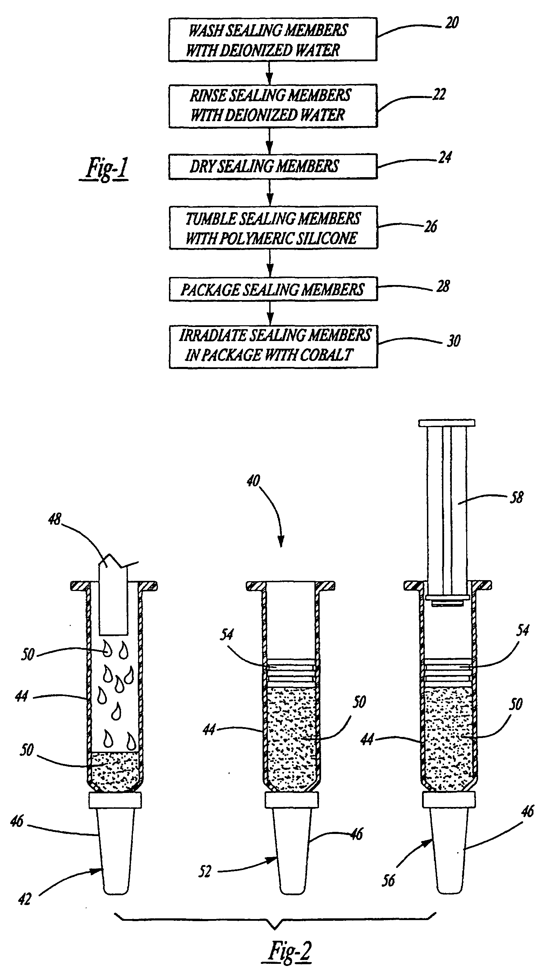

A flow chart of the method of this invention of lubricating a stopper member, which preferably is made from a rubber or elastomeric material, is provided in Figure 1. In step 20, the sealing members are washed. Preferably the sealing members are washed with hot deionized water. Most preferably, the sealing members are washed in deionized water at a temperature between 67 to 83°C (154°F and 181°F) for 1.5 minutes.

In step 22 the sealing members are rinsed. Preferably, the sealing members are rinsed in deionized water. Most preferably, the sealing members are rinsed in deionized water at a temperature between 67 and 83°C (154°F and 181°F) for 7.5 minutes.

In step 24 the sealing members are dried. Most preferably, the sealing members are dried for 30 minutes at 93.3°C (200°F).

In step 26, the sealing members are tumbled with polymeric silicone to coat the sealing members. Most preferably, the sealing members are tumbled with polymeric silicone for 60 minutes to coat the sealing members. A conventional tumbling device can be used.

In step 28, the coated sealing members are packaged in a container. Most preferably, the coated sealing members are packaged and sealed in the container. In step 30, the packaged and coated sealing members are irradiated. Most preferably, the packaged and coated sealing members are irradiated with Cobalt radiation at a target dose of 25 to 40 Joule per gram (2.5 to 4.0 Mrads). The radiation provides cross linking between the silicone molecules and adheres the silicone to the stopper. Thus, steps 20 through 30 produce a lubricated, sterile, sealing member. A significant advantage provided by the method of this invention is that the silicone does not become separated from the stopper and, therefore the likelihood of contaminating the medicament is greatly reduced.

This invention also includes forming a medicament receiving chamber from a plastic that has been mixed with a polymeric silicone to produce a pre-lubricated plastic material. The method preferably includes mixing a selected polymeric silicone with a plastic material during the compounding of the plastic material. Apart from the addition of the selected polymeric silicone, the compounding process is conventional. The polymeric silicone used has a viscosity of approximately 0.1 m2 per second (100,000 centistokes). The plastic material containing a polymeric silicone can be formed into a variety of drug delivery devices as will be described more fully below. Prelubrication of the plastic material further reduces the resistance between the medicament chamber interior wall and a coated sealing member produced according to the method described above. Moreover, prelubrication of the plastic material during the plastic compounding procedure effectively eliminates the possibility for the lubricant to become deposited in the medicament. Further, since the plastic chamber is prelubricated, the assembly/manufacturing step of applying a lubricant to the inside of the chamber is eliminated.

A schematic diagram of the processing steps of manufacturing a prefilled syringe using a lubricated sealing member designed according to the present invention is generally indicated at 40 in Figure 2. As shown at 42, a syringe 44 having a needle shield 46 receives a fill tube 48. The syringe 44 is made of plastic as described above. The fill tube 48 dispenses a medicament 50 into the syringe 44 to fill the syringe 44. In the stage illustrated at 52, a coated, lubricated, and irradiated sealing member 54, preferably made according to the method described above, is inserted into the syringe 44 in a fluid-tight relationship over the medicament 50. At 56, a plunger 58 is inserted into the sealing member 54. Put another way, step 42 involves filling the syringe 44 with a medicament 50, step 52 involves placing a lubricated sealing member 54 into the syringe 44, and step 56 involves connecting the plunger 58 and the sealing member 54. Of course, the sealing member 54 and plunger 58 can be preassembled before step 52 is performed.

The particular polymeric silicone used to lubricate the sealing member 54 preferably is selected in order to be compatible with the particular medicament in the drug delivery system and the material composition of the sealing member. In addition, it is necessary to comply with federal regulations regarding acceptable materials for use in a drug delivery system.

Polymeric silicones that may be used for this invention include: phenyl substitute silicones, vinyl substitute silicones, hydrogen substituted silicones, and others. One especially preferred silicone is known as Med-361, which is a polydimethyl siloxane, produced by Nusil and the most preferred viscosity of Med-361 is 100,000 centistokes. All of these silicones are used at a viscosity about 0.1 m2 per second (100,000 centistokes). Acceptable phenyl substituted silicones include: dimethyldiphenylpolysiloxane copolymers; dimethyl, methylphenylpolysiloxane copolymers; polymethylphenylsiloxane; and methylphenyl, dimethylsiloxane copolymers. The higher the phenyl content of the substituted silicone the lower the amount of radiation-induced crosslinking that occurs.

When compounding the silicone with a plastic, to form the chamber as described above, a silicone is used having a viscosity of 100,000 centistokes.

Vinyl substituted silicones that have been found to be advantageous for this invention include: vinyldimethyl terminated polydimethylsiloxanes; vinylmethyl, dimethylpolysiloxane copolymers; vinyldimethyl terminated vinylmethyl, dimethylpolysiloxane copolymers; divinylmethyl terminated polydimethylsiloxanes; polydimethylsiloxane, mono vinyl, mono n-butyldimethyl terminated; and vinylphenylmethyl terminated polydimethylsiloxanes. The vinyl substituted silicones also can be made in a variety of viscosities as noted above. Higher vinyl content provides more efficient radiation induced crosslinking.

The hydrogen substituted silicones that have been found to be advantageous for this invention include: dimethylhydro terminated polydimethylsiloxanes; methylhydro, dimethylpolysiloxanecopolymers; methylhydro terminated methyloctyl siloxane copolymers; and methylhydro, phenylmethyl siloxane copolymers. The hydrogen substituted siloxanes can be used in a variety of viscosities as noted above.

Other substituted silicones that may be used in the method of this invention include: polyfluoroalkylmethyl siloxanes; fluoralkyl, dimethyl siloxanecopolymers; and polymethylalkylsiloxanes.

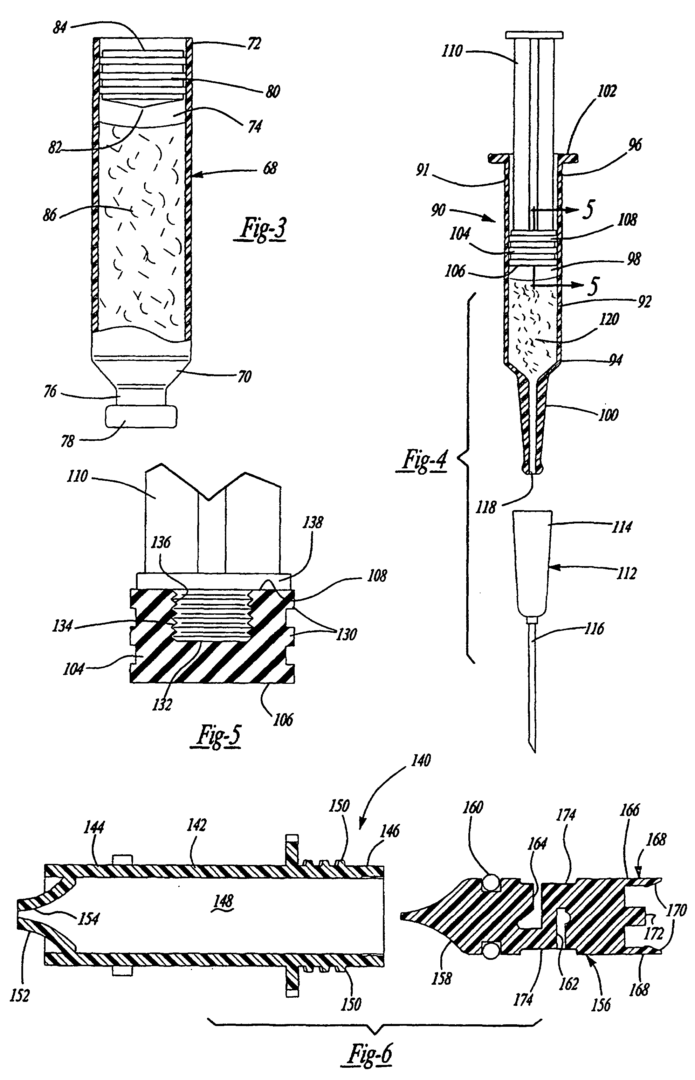

Figures 3 through 6 illustrate example drug delivery assemblies that incorporate a lubricated sealing member and a medicament chamber made according to this invention. A medicament cartridge is shown generally at 66 in Figure 3, which may be made from prelubricated plastic. The medicament cartridge 66 comprises a generally cylindrical barrel 68 having a first end 70, a second end 72, and an interior chamber 74. A neck portion 76 is located adjacent the first end 70. A seal 78 surrounds an end of the neck portion 76 and seals the neck portion 76. A lubricated stopper 80, made according to the method described above, is received in a fluid-tight relationship into the interior chamber 74 through the second end 72 of the medicament cartridge 66. The stopper 80 includes a first side 82 and a second side 84. A medicament 86 is located between the first side 82 of the stopper 80 and the seal 78. As will be understood by those skilled in the art, such medicament cartridges 66 are designed to be received in a wide variety of delivery devices (not shown). The delivery devices include a needle cannula for penetrating the seal 78 and a plunger mechanism for moving the stopper 80 from the second end 72 toward the first end 70 to expel the medicament 86 from the interior chamber 74 during an injection.

An exploded side view of a syringe and a needle cannula is generally indicated at 90 in Figure 4. The syringe 91 includes a cylindrical barrel 92 having a first end 94 and a second end 96 and an interior chamber 98. A neck portion 100 is located adjacent the first end 94. A flange 102 is located adjacent the second end 96. A lubricated stopper 104, formed according to the method of this invention is received in a fluid-tight relationship into the interior chamber 98. The stopper 104 has a first side 106 and a second side 108. A plunger 110 is received in the second side 108 of the stopper 104. A needle cannula 112 includes a hub 114 and a needle 116. The neck portion 100 includes a fluid channel 118. A medicament 120 is located in the interior chamber 98 between the first side 106 of the stopper 104 and the neck portion 100. The needle cannula 112 is received on the neck portion 100. The fluid channel 118 is in fluid communication with the needle 116.

Figure 5 is a cross-sectional side view of a portion of the plunger 110 and the lubricated stopper 104. The stopper 104 preferably includes a plurality of ribs 130. An interior space 132 extends from the second side 108 of the stopper 104 into the stopper 104. A set of internal threads 134 lines the interior space 132. A set of external threads 136 are located on the plunger 110 adjacent a plunger base 138. The internal threads 134 are adapted to receive the external threads 136 to secure the plunger 110 to the stopper 104 so the two will move in unison.

An exploded side view of a cartridge for use with a needleless injector is shown at 140 in Figure 6. The cartridge 140 includes a cylindrical barrel 142 having a first end 144, a second end 146, and an interior chamber 148. A luer lock arrangement 150 preferably is located adjacent the second end 146 for securing the cartridge 140 into a needleless injector. A tapered tip 152 is located adjacent the first end 144 and includes a fluid orifice 154.

A plunger 156 is slidably received in the interior chamber 148. The plunger 156 includes a tip portion 158 and a lubricated sealing member 160, formed according to the method of this invention, adjacent the tip portion 158. The sealing member 160 is in a fluid-tight relationship with the interior chamber 148 when the plunger 156 is received into the chamber 148. Preferably, the sealing member 160 is an O-ring. The plunger 156 further includes a first cutout 162 and a second cutout 164. A plunger portion 166 includes a series of spaced tabs 168 that facilitate cooperation between an injector driver member (not shown) and the plunger 156. A tab lip 170 is located on each of the spaced tabs 168. A boss 172 is located centrally to the spaced tabs 168. The plunger 156 further includes a pair of slots 174.

As will be understood by those skilled in the art, the needleless injector cartridge 140 is designed to be utilized with a variety of commercially available injector devices (not shown). The injector device driver mechanism is used to drive the plunger 156 from a position adjacent the second end 146 toward the first end 144 and expel a medicament (not shown) out of the interior chamber 148, through the fluid orifice 154 to accomplish a needleless injection.

As will be understood by those skilled in the art, all of the sealing members and stoppers are in fluid-tight relationship with the walls of the interior chambers. The sealing members and stoppers preferably are made of rubber or elastomeric materials. The specific embodiments described above are for illustration purposes only. A plurality of applications or uses for the lubrication methods of this invention have been shown.

The foregoing description is exemplary rather than limiting in nature. Variations and modifications to the disclosed embodiments may become apparent to those skilled in the art that still come within the scope of the claims. Accordingly, the scope of legal protection afforded this invention can only be determined by studying the following claims.

## Claims

1. A medicament delivery device comprising:
a chamber body (44) having an interior surface, said chamber body (44) being made from a plastic material that includes a first polymeric silicone mixed into the plastic material wherein said first polymeric silicone has a viscosity of approximately 100,000 x 10⁻⁶ m²/s (100,000 centistokes).

2. The device of claim 1, further comprising a sealing member (54) slidably received within said chamber (44) such that an exterior surface on said sealing member (54) sealingly engages said chamber interior surface.

3. The device of claim 2, wherein said sealing member (54) outer surface includes a second polymeric silicone adhered to said surface by crosslinking bonds formed between molecules of said second polymeric silicone.

4. The device of claim 3 wherein the second polymeric silicone has a viscosity of 1,000 to 100,000 x 10⁻⁶ m²/s (1,000 to 100,000 centistokes).

5. The device of claim 1, wherein said first polymeric silicone comprises a polydimethyl siloxane.

6. The device of claim 1 wherein the first polymeric silicone is one of the group of vinyl substituted silicones comprising vinyldimethyl terminated polydimethylsiloxanes; vinylmethyl, dimethylpolysiloxane copolymers; vinyldimethyl terminated vinylmethyl, dimethylpolysiloxane copolymers; divinylmethyl terminated polydimethylsiloxanes; polydimethylsiloxane, mono vinyl, mono n-butyldimethyl terminated; and vinylphenylmethyl terminated polydimethylsiloxanes.

7. The device of claim 1 wherein the first polymeric silicone is one of the group of hydrogen substituted silicones comprising dimethylhydro terminated polydimethylsiloxanes; methylhydro, dimethylpolysiloxanecopolymers; methylhydro terminated methyloctyl siloxane copolymers; and methylhydro phenylmethyl siloxane copolymers.

8. The device of claim 1 wherein the first polymeric silicone is selected from polyfluoroalkylmethyl siloxanes; fluoroalkyl, dimethyl siloxane copolymers; and polymethylalkylsiloxanes.

9. A method for making a medicament delivery device, comprising the steps of:
(A) providing a chamber body (44) according to claim 1 having an interior surface, adapted to be in contact with a medicament (50), said chamber body (44) being made from a plastic material that includes a first polymeric silicone mixed into the plastic material, wherein the first polymeric silicone has a viscosity of approximately 0.1 m²/s (100,000 centistokes);
(B) providing a sealing member (54) sildably received in the chamber (44) such that an exterior surface on the sealing member (54) sealingly engages the chamber (44) interior surface;
(C) cotaining the sealing member (54) with a second polymeric silicone having a plurality of polymeric molecules; and
(D) irradiating the coated sealing member (54) to thereby adhere the second polymeric silicone to the sealing member (54).

10. The method of claim 9, including the steps of washing the sealing member (54) in deionized water; rinsing the sealing member (54) in deionized water; and drying the sealing member (54) prior to coating the outer surface.

11. The method of claim 9, including using deionized water having a temperature in the range from 65.5 to 82.2°C (150 degrees F to 180 degrees F).

12. The method of claim 9, including drying the sealing member (54) using a temperature of 93.3°C (200 degrees F).

13. The method of claim 9, wherein step (A) includes tumbling the sealing member (54) and the second polymeric silicone.

14. The method of claim 9, wherein step (D) includes placing the sealing member (54) in a sealed package and irradiating the sealing member (54) and the sealed package using radiation levels in the range from 25 to 40 Joule/gram (2.5 to 4.0 Mrads).

15. The method of claim 9, wherein step (D) is performed using Cobalt radiation.

16. The method of claim 9, wherein the second polymeric silicone has a viscosity in the range from 1,000 to 100,000 x 10⁻⁶ m²/s (1,000 to 100,000 centistokes).

17. The method of claim 9, wherein said second silicone comprises a polydimethyl siloxane.

18. The method of claim 9, wherein said second silicone is one of the group of phenyl substituted silicones consisting of dimethyldiphenylpolysiloxane copolymers; dimethylmethylphenylpolysiloxane copolymers; polymethylphenylsiloxane; and methylphenyl dimethylsiloxane copolymers.

19. The method of claim 9, wherein the sealing member (54) is received in the chamber (44) without any external lubricant being placed on an interior surface of the chamber (44).

## Patentansprüche

1. Medikamentenabgabevorrichtung mit:
einem Kammerkörper (44) mit einer Innenfläche, wobei der Kammerkörper (44) aus einem Kunststoffmaterial gefertigt ist, das ein in das Kunststoffmaterial eingemischtes erstes polymeres Silicon aufweist, welches eine Viskosität von ungefähr 100.000 x 10⁻⁶ m²/s (100.000 Centistokes) hat.

2. Vorrichtung nach Anspruch 1, ferner mit einem Dichtelement (54), das gleitbar derart in der Kammer (44) aufgenommen ist, dass eine Außenfläche des Dichtelements (54) dichtend an der Kammerinnenfläche angreift.

3. Vorrichtung nach Anspruch 2, bei der die Außenfläche des Dichtelements (54) ein zweites polymeres Silicon aufweist, das aufgrund von Vernetzungen zwischen Molekülen des zweiten polymeren Silicons an dieser Fläche haftet.

4. Vorrichtung nach Anspruch 3, bei der das zweite polymere Silicon eine Viskosität von 1.000 bis 100.000 x 10⁻⁶ m²/s (1.000 bis 100.000 Centistokes) aufweist.

5. Vorrichtung nach Anspruch 1, bei der das erste polymere Silicon ein Polydimethylsiloxan aufweist.

6. Vorrichtung nach Anspruch 1, bei der das erste polymere Silicon ein Silicon aus der Gruppe von vinylsubstituierten Siliconen ist, die umfassen: Polydimethylsiloxane mit Vinyldimethyl-Endgruppe; Vinylmethyl-Dimethylpolysiloxan-Copolymere; Vinylmethyl-Dimethylpolysiloxan-Copolymere mit Vinyldimethyl-Endgruppe; Polydimethylsiloxane mit Divinylmethyl-Endgruppe; Polydimethylsiloxan-Monovinyl mit Mono-n-Butyldimethyl-Endgruppe; und Polydimethylsiloxane mit Vinylphenylmethyl-Endgruppe.

7. Vorrichtung nach Anspruch 1, bei der das erste polymere Silicon ein Silicon aus der Gruppe von wasserstoffsubstituierten Siliconen ist, die umfassen: Polydimethylsiloxane mit Dimethylhydro-Endgruppe; Methylhydro-Dimethylpolysiloxan-Copolymere; Methyloctylsiloxan-Copolymere mit Methylhydro-Endgruppe; und Methylhydro-Phenylmethylsiloxan-Copolymere.

8. Vorrichtung nach Anspruch 1, bei der das erste polymere Silicon aus Polyfluoralkylmethylsiloxanen; Fluoralkyl-Dimethylsiloxan-Copolymeren; und Polymethylalkylsiloxanen ausgewählt ist.

9. Verfahren zum Herstellen einer Medikamentenabgabevorrichtung, mit folgenden Schritten:
(A) Bereitstellen eines Kammerkörpers (44) nach Anspruch 1 mit einer Innenfläche, der mit einem Medikament (50) in Kontakt steht, wobei der Kammerkörper (44) aus einem Kunststoffmaterial gefertigt ist, das ein in das Kunststoffmaterial eingemischtes erstes polymeres Silicon aufweist, wobei das erste polymere Silicon eine Viskosität von ungefähr 0,1 m²/s (100.000 Centistokes) hat;
(B) Bereitstellen eines Dichtelements (54), das derart gleitbar in der Kammer (44) aufgenommen ist, dass eine Außenfläche des Dichtelements (54) dichtend an der Innenfläche der Kammer (44) angreift;
(C) Beschichten des Dichtelements (54) mit einem zweiten polymeren Silicon mit mehreren Polymermolekülen; und
(D) Bestrahlen des beschichteten Dichtelements (54) derart, dass das zweite polymere Silicon an dem Dichtelement (54) haftet.

10. Verfahren nach Anspruch 9, mit den Schritten des Waschens des Dichtelements (54) in entionisiertem Wasser; Spülens des Dichtelements (54) in entionisiertem Wasser; und Trocknens des Dichtelements (54) vor dem Beschichten der Außenfläche.

11. Verfahren nach Anspruch 9, mit dem Schritt der Verwendung von entionisiertem Wasser mit einer Temperatur im Bereich von 65,5 bis 82,2 °C (150 °F bis 180 °F).

12. Verfahren nach Anspruch 9, mit dem Schritt des Trocknens des Dichtelements (54) bei einer Temperatur von 93,3 °C (200 °F).

13. Verfahren nach Anspruch 9, bei dem Schritt (A) das Trommelbeschichten des Dichtelements (54) mit dem zweiten polymeren Silicon umfasst.

14. Verfahren nach Anspruch 9, bei dem Schritt (D) das Platzieren des Dichtelements (54) in eine versiegelte Verpackung und das Bestrahlen des Dichtelements (54) und der versiegelten Verpackung mit Bestrahlungspegeln im Bereich von 25 bis 40 Joule/Gramm (2,5 bis 4,0 Mrad) umfasst.

15. Verfahren nach Anspruch 9, bei dem in Schritt (D) eine Cobaltbestrahlung durchgeführt wird.

16. Verfahren nach Anspruch 9, bei dem das zweite polymere Silicon eine Viskosität im Bereich von 1.000 bis 100.000 x 10⁻⁶ m²/s (1.000 bis 100.000 Centistokes) aufweist.

17. Verfahren nach Anspruch 9, bei dem das zweite Silicon ein Polydimethylsiloxan enthält.

18. Verfahren nach Anspruch 9, bei dem das zweite Silicon einer Gruppe von phenylsubstituierten Siliconen bestehend aus Dimethyldiphenylpolysiloxan-Copolymeren; Dimethyl-Methylphenylpolysiloxan-Copolymeren; Polymethylphenylsiloxan; und Methylphenyl-Dimethylsiloxan-Copolymeren angehört.

19. Verfahren nach Anspruch 9, bei dem das Dichtelement (54) ohne das Aufbringen eines externen Schmiermittels auf eine Innenfläche der Kammer (44) in der Kammer (44) aufgenommen wird.

## Revendications

1. Dispositif de fourniture de médicaments comprenant :
un corps de chambre (44) ayant une surface intérieure, ledit corps de chambre (44) étant constitué d'une matière plastique qui comprend une première silicone polymère mélangée dans le matériau en matière plastique, dans lequel ladite première silicone polymère présente une viscosité d'environ 100 000 x 10⁻⁶m²/s (100 000 centistokes).

2. Dispositif selon la revendication 1, comprenant, en outre, un élément d'étanchéité (54) reçu de façon coulissante dans ladite chambre (44) de sorte qu'une surface extérieure sur ledit élément d'étanchéité (54) vienne en contact d'étanchéité avec ladite surface intérieure de la chambre.

3. Dispositif selon la revendication 2, dans lequel ladite surface extérieure de l'élément d'étanchéité (54) comprend une deuxième silicone polymère adhérant à ladite surface par des liaisons de réticulation formées entre des molécules de ladite deuxième silicone polymère.

4. Dispositif selon la revendication 3, dans lequel la deuxième silicone polymère présente une viscosité de 1 000 à 100 000 x 10⁻⁶ m²/s (1 000 à 100 000 centistokes).

5. Dispositif selon la revendication 1, dans lequel ladite première silicone polymère comprend un polydiméthylsiloxane.

6. Dispositif selon la revendication 1, dans lequel la première silicone polymère est une du groupe des silicones à substitution vinyle comprenant les polydiméthylsiloxanes à terminaison vinyldiméthyle, les copolymères vinylméthyl-diméthylpolysiloxane, les copolymères vinylméthyldiméthyl-polysiloxane, à terminaison vinyldiméthyle, les polydiméthyl-siloxanes à terminaison divinylméthyle, le polydiméthylsiloxane à terminaison mono vinyle, mono n butyldiméthyle et les polydiméthylsiloxanes à terminaison vinylphénylméthyle.

7. Dispositif selon la revendication 1, dans lequel la première silicone polymère est une du groupe comprenant les silicones substituées par de l'hydrogène comprenant les poly-diméthylsiloxanes à terminaison diméthylhydro, les copolymères méthylhydrodiméthylpolysiloxane, les copolymères méthyloctyl siloxane à terminaison méthylhydro et les copolymères méthylhydro phénylméthyl siloxane.

8. Dispositif selon la revendication 1, dans lequel la première silicone polymère est choisie parmi les polyfluoro-alkylméthyl siloxanes, les copolymères fluoroalkyl diméthyl siloxane et les polyméthylalkylsiloxanes.

9. Procédé d'obtention d'un dispositif de fourniture de médicaments, comprenant les étapes de :
(A) fournir un corps de chambre (44) selon la revendication 1, ayant une surface intérieure agencée pour être en contact avec un médicament (50), ledit corps de chambre (44) étant constitué d'un matériau en matière plastique qui comprend une première silicone polymère mélangée dans la matière plastique, dans lequel la première silicone polymère présente une viscosité d'environ 0,1 m²/s (100 000 centistokes) ;
(B) fournir un élément d'étanchéité (54) reçu de façon coulissante dans la chambre (44) de sorte qu'une surface extérieure sur l'élément d'étanchéité (54) vienne en contact d'étanchéité avec la surface intérieure de la chambre (44) ;
(C) enrober l'élément d'étanchéité (54) avec une deuxième silicone polymère ayant une pluralité de molécules polymères ; et
(D) irradier l'élément d'étanchéité enrobé (54) pour faire adhérer ainsi la deuxième silicone polymère à l'élément d'étanchéité (54).

10. Procédé selon la revendication 9, comprenant les étapes de lavage de l'élément d'étanchéité (54) dans de l'eau désionisée, de rinçage de l'élément d'étanchéité (54) dans de l'eau désionisée et de séchage de l'élément d'étanchéité (54) avant l'enrobage de la surface extérieure.

11. Procédé selon la revendication 9, comprenant l'utilisation d'eau désionisée à une température dans la gamme de 65,5 à 82,2 °C (150 °F à 180 °F).

12. Procédé selon la revendication 9, comprenant le séchage de l'élément d'étanchéité (54) en utilisant une température de 93,3 °C (200 °F).

13. Procédé selon la revendication 9, dans lequel l'étape (A) comprend l'agitation de l'élément d'étanchéité (54) et de la deuxième silicone polymère.

14. Procédé selon la revendication 9, dans lequel l'étape (D) comprend la mise en place de l'élément d'étanchéité (54) dans un conditionnement étanchéifié et l'irradiation de l'élément d'étanchéité (54) et du conditionnement étanchéifié en utilisant des niveaux de rayonnement dans la gamme de 25 à 40 joules/gramme (2,5 à 4,0 Mrads).

15. Procédé selon la revendication 9, dans lequel l'étape (D) est réalisée en utilisant une irradiation de cobalt.

16. Procédé selon la revendication 9, dans lequel la deuxième silicone polymère présente une viscosité dans la gamme de 1 000 à 100 000 x 10⁻⁶ m²/s (1 000 à 100 000 centistokes).

17. Procédé selon la revendication 9, dans lequel ladite deuxième silicone comprend un polydiméthylsiloxane.

18. Procédé selon la revendication 9, dans lequel la deuxième silicone est une du groupe des silicones à substitution phényle comprenant les copolymères diméthyldiphénylpoly-siloxane, les copolymères diméthylméthylphénylpolysiloxane, les copolymères polyméthylphénylsiloxane et méthylphényl-diméthylsiloxane.

19. Procédé selon la revendication 9 dans lequel l'élément d'étanchéité (54) est reçu dans la chambre (44) sans aucun lubrifiant externe placé sur la surface intérieure de la chambre (44).
